(19) Europäisches Patentamt
European Patent Office
Office européen des brevets

(11) Veröffentlichungsnummer: **0 230 254 B1**

(12) # EUROPÄISCHE PATENTSCHRIFT

(45) Veröffentlichungstag der Patentschrift: **04.07.90**

(51) Int. Cl.[5]: **C07C 27/12**, C07C 27/26, C07C 49/403, C07C 35/08, C07C 409/06, C07C 45/33, C07C 45/53, C07C 29/50, C07C 45/80

(21) Anmeldenummer: **87100352.1**

(22) Anmeldetag: **13.01.87**

(54) **Verfahren zur Aufarbeitung von Cyclohexylhydroperoxid, Cyclohexanol und Cyclohexanon enthaltenden Reaktionsgemischen.**

(30) Priorität: **17.01.86 DE 3601218**

(43) Veröffentlichungstag der Anmeldung: **29.07.87 Patentblatt 87/31**

(45) Bekanntmachung des Hinweises auf die Patenterteilung: **04.07.90 Patentblatt 90/27**

(84) Benannte Vertragsstaaten: **BE CH DE FR GB IT LI NL**

(56) Entgegenhaltungen:
**EP-A- 0 092 867**
**FR-A- 2 087 365**
**FR-A- 2 140 088**
**GB-A- 1 159 006**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Hartig, Jürgen, Dr., In der Schleit 9, D-6718 Gruenstadt(DE)**
Erfinder: **Herrmann, Guenter, Dr., Haeusserstrasse 51, D-6900 Heidelberg(DE)**
Erfinder: **Lucas, Ekhart, Dr., Burgunderweg 7, D-6706 Wachenheim(DE)**

**EP 0 230 254 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Bei der Oxidation von Cyclohexan mit Luft oder Sauerstoff unter erhöhtem Druck und erhöhter Temperatur erhält man Gemische aus Cyclohexylhydroperoxid, Cyclohexanol und Cyclohexanon, anderen Peroxiden, Säuren und Estern. Zur Verbesserung der Ausbeute wird üblicherweise das gebildete Cyclohexylhydroperoxid unter besonderen Bedingungen zu Cyclohexanol und Cyclohexanon umgesetzt. In vielen Fällen wird durch Zugabe von speziellen Übergangsmetallsalzen während der Oxidation die gleichzeitige Zersetzung des gebildeten Cyclohexylhydroperoxids bewerkstelligt. So wird in der DE-PS 1 002 754 die Oxidation und die Deperoxidation mit homogen gelöstem Kobalt oder Chromsalzen beschrieben. Auch Kupfer und Mangansalze erfüllen diese Aufgabe, wie aus der DE-AS 11 93 501 zu entnehmen ist. Diese Verfahren sind jedoch hinsichtlich der Ausbeute verbesserungsbedürftig. Dies umso mehr als unter den Bedingungen der Cyclohexanoxidation erhebliche Mengen an unerwünschten Nebenprodukten entstehen. Aus der EP-Anmeldung 92 867 ist auch bereits ein Verfahren bekannt, bei dem man die Zersetzung von Cyclohexylhydroperoxid in Cyclohexanoxidationsgemischen durch Zusatz von Kobaltsalze enthaltender Alkalilauge bei einer Temperatur von 110°C durchführt. Die hierbei anfallenden Ablaugen sind nicht mehr verwendungsfähig und müssen entsorgt werden.

Es war deshalb die technische Aufgabe gestellt, die Zersetzung von Cyclohexylhydroperoxid so vorzunehmen, daß möglichst wenig Nebenprodukte entstehen, die Zersetzung bei einer möglichst niedrigen Temperatur rasch verläuft, keine Ablagerungen auftreten und zudem die anfallende Ablauge wieder der technischen Verwertung zugeführt wird.

Diese Aufgabe wird gelöst in einem Verfahren zur Aufarbeitung von Cyclohexylhydroperoxid, Cyclohexanol und Cyclohexanon enthaltenden Reaktionsgemischen, die durch Oxidation von Cyclohexan mit molekularem Sauerstoff oder solchen enthaltenden Gasen in flüssiger Phase bei einer Temperatur von 130 bis 200°C und unter einem Druck von 5 bis 25 bar erhalten wurden, durch Behandeln mit wäßrigen Alkalilösungen in Gegenwart von Kobaltsalzen bei erhöhter Temperatur, dadurch gekennzeichnet, daß man Phosphonsäureverbindungen der Formel

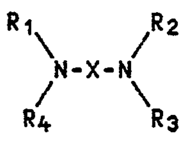

I,

in der X einen Alkylenrest mit 2 bis 6 Kohlenstoffatomen bedeutet und $R_1$ und $R_4$ die Gruppe $-CH_2PO_3H$ bezeichnet und $R_1$, $R_2$ und $R_3$ gleich oder verschieden sein können und jeweils die Gruppen $-CH_2PO_3H$, $-CH_2OH$ oder ein Wasserstoffatom oder einen Alkylrest mit 1 bis 4 Kohlenstoffatomen bezeichnen, mitverwendet.

Das neue Verfahren hat den Vorteil, daß die gezielte Zersetzung des Cyclohexylhydroperoxids zu Cyclohexanol und Cyclohexanon bereits bei relativ niedriger Temperatur rasch verläuft. Ferner hat das neue Verfahren den Vorteil, daß wenig Nebenprodukte gebildet werden und keine Ablagerungen eintreten. Schließlich hat das neue Verfahren den Vorteil, daß Alkalicarbonatlösungen verwendet werden können, die auf einfache Weise wieder aufgearbeitet und in das Verfahren zurückgeführt werden können.

Erfindungsgemäß geht man von Cyclohexylhydroperoxid, Cyclohexanol und Cyclohexanon enthaltenen Reaktionsgemischen aus, die durch Oxidation von Cyclohexan mit molekularem Sauerstoff oder solchen enthaltenen Gasen, z.B. Luft in flüssiger Phase bei einer Temperatur von 130 bis 200°C und zweckmäßig unter Drücken von 5 bis 25 bar erhalten wurden. Solche Gemische enthalten in der Regel 0,5 bis 10 Gew.-% Cyclohexylhydroperoxid. Vorteilhaft werden die so erhaltenen Reaktionsgemische vor der Weiterbehandlung mit Wasser oder Alkalilaugen gewaschen. Typische Reaktionsgemische enthalten neben Cyclohexan 1 bis 7 Gew.-% Cyclohexanon und Cyclohexanol sowie 0,5 bis 3,5 Gew.-% Cyclohexylhydroperoxid, ferner Nebenprodukte, wie Ester, Carbonsäuren sowie gegebenenfalls Wasser, z.B. bis zu 2 Gew.-%. Geeignete Reaktionsgemische erhält man beispielsweise nach dem in der DE-PS 1 046 610 beschriebenen Verfahren.

Das Reaktionsgemisch wird mit wäßrigen Alkalilösungen, z.B. Lösungen von Natrium- oder Kaliumhydroxid oder der entsprechenden Carbonate, behandelt. Falls man Alkalihydroxidlösungen verwendet, eignen sich vorteilhaft 5- bis 50-gew.%ige, insbesondere 20- bis 30-gew.%ige wäßrige Lösungen. Sofern Alkalicarbonatlösungen verwendet werden, wendet man diese als 5- bis 30-gew.%ige, insbesondere 20 bis 25-gew.%ige wäßrige Lösungen an. Besonders bevorzugt wird Natriumcarbonat verwendet. In der Regel wendet man je kg Reaktionsgemisch 3 bis 10 g Alkalihydroxid oder -carbonat in Form einer wäßrigen Lösung an.

Die Behandlung wird in Gegenwart von Kobaltsalzen, vorzugsweise wasserlöslichen Kobaltsalzen durchgeführt. Geeignete Salze sind vorteilhaft wasserlösliche Nitrate, Sulfate, insbesondere Salze von niederen Fettsäuren, wie Acetate. Vorteilhaft wendet man die genannten Kobaltsalze in einer Menge von 0,1 bis 1000 ppm, insbesondere 1 bis 100 ppm, berechnet als Kobalt und bezogen auf die wäßrige Alkalilösung, an.

Es ist ein erfindungswesentliches Merkmal, daß man zusätzlich die Umsetzung in Gegenwart von Phosphonsäuren der Formel

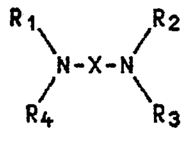

I,

in der X einen Alkylenrest mit 2 bis 6 Kohlenstoffatomen bedeutet und $R_1$ und $R_4$ die Gruppe $-CH_2PO_3H$ bezeichnet und $R_1$, $R_2$ und $R_3$ gleich oder verschieden sein können und jeweils die Gruppen $-CH_2PO_3H$, $-CH_2OH$ oder ein Wasserstoff-

atom oder einen Alkylrest mit 1 bis 4 Kohlenstoffatomen bezeichnen, durchführt. Geeignete Verbindungen sind beispielsweise Ethylendiamintetramethylenphosphonsäure, 1,3-Propylendiamintetramethylenphosphonsäure, 1,4-Butylendiamintetramethylenphosphonsäure, Ethylendiamindimethyldimethylenphosphonsäure, 1,3-Propylendiamindimethyldimethylenphosphonsäure, Ethylendiamin-methyl-trimethylenphosphonsäure. Besonders bevorzugt sind Ethylendiamintetramethylenphosphonsäure und 1,3-Propylendiamintetramethylenphosphonsäure. Der Alkylenrest X kann geradkettig oder verzweigt sein und hat vorzugsweise 2 oder 3 Kohlenstoffatome.

Vorteilhaft wendet man Phosphonsäuren der Formel I in einer Menge an, daß die mitverwendeten Metallverbindungen als lösliche Komplexverbindungen in der wäßrigen Alkalilösung vorliegen. Es hat sich bewährt, die Verbindungen der Formel I in Mengen von 10 bis 2000 ppm, bezogen auf die wäßrige Alkalilösung, mitzuverwenden.

Die Behandlung wird vorteilhaft bei einer Temperatur von 50 bis 150°C, insbesondere bei einer Temperatur von 80 bis 120°C, durchgeführt. Hierbei wendet man z.B. einen Druck von 1 bis 50 bar an. Zweckmäßig richtet sich der Druck nach der angewandten Temperatur. Die Behandlung des Cyclohexanoxidationsgemisches wird zweckmäßig im Gleichstrom oder Gegenstrom, besonders bevorzugt im Gleichstrom, durchgeführt. Die Behandlungsdauer beträgt in der Regel von 2 bis 60 Minuten. Nach erfolgter Behandlung wird die wäßrige Alkalilösung mit üblichen Methoden, z.B. Dekantieren, abgetrennt und aus der organischen Phase Cyclohexan abdestilliert und Cyclohexanol und Cyclohexanon gewonnen. Das zurückgewonnene Cyclohexan wird wieder der Oxidation zugeführt. Die erhaltene wäßrige Ablauge, die nunmehr neben Alkali gelöste Alkalisalze von niederen Fettsäuren enthält, wird vorteilhaft verbrannt und Alkalicarbonat zurückgewonnen. Besonders bewährt hat sich diese Arbeitsweise bei der Verwendung von Natriumcarbonat. Das bei der Verbrennung anfallende Natriumcarbonat kann wiederum als wäßrige Lösung für die erfindungsgemäße Behandlung von Oxidationsgemischen aus der Cyclohexanoxidation verwendet werden. Somit entfällt eine Entsorgung der behandlungsbedürftigen Ablaugen.

Cyclohexanol und Cyclohexanon sind wichtige Ausgangsverbindungen für Faserrohstoffe, wie Adipinsäure oder Caprolactam.

Das Verfahren nach der Erfindung sei an folgenden Beispielen veranschaulicht.

Beispiel 1

100 g eines Cyclohexanoxidationsgemisches, das neben Cyclohexan 1,7 Gew.-% Cyclohexanol, 0,3 Gew.-% Cyclohexanon, 1,2 Gew.-% Cyclohexylhydroperoxid sowie Nebenprodukte enthält, wird mit 100g einer 20-gew.%igen Natriumcarbonatlösung, die 0,13 g Ethylendiamintetramethylenphosphonsäure und 5 mg Kobalt als Kobaltacetat gelöst enthält, eine Stunde unter Rühren auf 80°C erhitzt. Nach der Behandlung ist das Cyclohexylhydroperoxid zu 100% zersetzt.

Beispiel 2

Eine 2 m lange von außen beheizbare pulsierte Siebbodenkolonne mit einem Innendurchmesser von 25 mm wird im Gleichstrom bei einer Temperatur von 75°C mit einem Cyclohexanoxidationsgemisch, das neben Cyclohexan 1,7 Gew.-% Cyclohexanol, 0,9 Gew.-% Cyclohexanon, 1,2 Gew.-% Cyclohexylhydroperoxid sowie Nebenprodukte enthält, und einer 10-gew.%igen Natriumcarbonatlösung, die 25 ppm Kobalt in Form eines Komplexes mit Ethylendiamintetramethylenphosphonsäure enthält, beaufschlagt. Stündlich führt man 1 l des Oxidationsgemisches und 0,2 l Natriumcarbonatlösung zu. Die Verweilzeit beträgt 50 Minuten. Nach Trennen in eine organische und wäßrige Phase wird die organische Phase abgetrennt und analysiert. Das in der organischen Phase enthaltene Cyclohexylhydroperoxid wurde zu 95 bis 98% umgesetzt.

Vergleichsbeispiel 1

Man verfährt wie in Beispiel 1 beschrieben, verwendet jedoch lediglich 100 g 20-gew.%ige Natriumcarbonatlösung, die nur 5 mg Kobaltacetat, berechnet als Kobalt, enthält. Im übrigen verfährt man wie in Beispiel 1 beschrieben. Eine Analyse des so behandelten Cyclohexanoxidationsgemisches ergab, daß weniger als 2 % des Cyclohexylhydroperoxids zersetzt wurden.

Vergleichsbeispiel 2

Man verfährt wie in Beispiel 1 beschrieben und verwendet in 3 getrennten Ansätzen

a) 100 g 20-gew.%ige Natriumcarbonatlösung mit 1000 ppm Vanadium als Vanadiumpentoxid,
b) 1000 ppm Kobalt als Natriumhexanitrokobaltat,
c) 1000 ppm Chrom als Chromtrioxid.

Nach analoger Arbeitsweise wie in Beispiel 1 beschrieben wird die Zersetzung des Cyclohexylhydroperoxids bestimmt. Diese beträgt in a) < 2 %, in b) < 2 % und in c) < 2 %.

**Patentansprüche**

1. Verfahren zur Aufarbeitung von Cyclohexylhydroperoxid, Cyclohexanol und Cyclohexanon enthaltenden Reaktionsgemischen, die durch Oxidation von Cyclohexan mit molekularem Sauerstoff oder solchen enthaltenden Gasen in flüssiger Phase bei einer Temperatur von 130 bis 200°C und unter einem Druck von 5 bis 25 bar erhalten wurden, durch Behandeln mit wäßrigen Alkalilösungen in Gegenwart von Kobaltsalzen bei erhöhter Temperatur, dadurch gekennzeichnet, daß man Phosphonsäureverbindungen der Formel

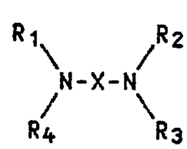

I,

in der X einen Alkylenrest mit 2 bis 6 Kohlenstoffatomen bedeutet und $R_1$ und $R_4$ die Gruppe $-CH_2PO_3H$ bezeichnen und $R_2$ und $R_3$ gleich oder verschieden sein können und jeweils die Gruppen $-CH_2PO_3H$, $-CH_2OH$ oder ein Wasserstoffatom oder einen Alkylrest mit 1 bis 4 Kohlenstoffatomen bezeichnet, mitverwendet.

2. Verfahren nach Anspruch 1, dadurch gekennzeichnet, daß man wasserlösliche Kobaltsalze mitverwendet.

3. Verfahren nach den Ansprüchen 1 und 2, dadurch gekennzeichnet, daß man Ethylendiamintetramethylenphosphonsäure verwendet.

4. Verfahren nach den Ansprüchen 1 bis 3, dadurch gekennzeichnet, daß man wäßrige Natriumcarbonatlösung verwendet.

5. Verfahren nach den Ansprüchen 1 bis 4, dadurch gekennzeichnet, daß man die Ablauge nach der Behandlung abtrennt, verbrennt und das so gewonnene Natriumcarbonat wiederum als wäßrige Lösung für die Behandlung von Cyclohexanoxidationsgemischen verwendet.

## Claims

1. A process for working up a reaction mixture which contains cyclohexyl hydroperoxide, cyclohexanol and cyclohexanone and is obtained by oxidizing cyclohexane with molecular oxygen, or a gas containing this, in the liquid phase at from 130 to 200°C and under from 5 to 25 bar, by treatment with an aqueous alkali solution in the presence of a cobalt salt at elevated temperatures, wherein a phosphonic acid compound of the formula

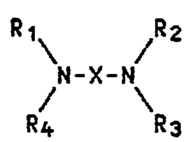

I

where X is alkylene of 2 to 6 carbon atoms, $R_1$ and $R_4$ are $-CH_2PO_3H$ and $R_2$ and $R_3$ may be identical or different and are each $-CH_2PO_3H$, $-CH_2OH$, hydrogen or alkyl of 1 to 4 carbon atoms, is concomitantly used.

2. A process as claimed in claim 1, wherein a watersoluble cobalt salt is concomitantly used.

3. A process as claimed in either of claims 1 and 2, wherein ethylenediaminetetramethylenephosphonic acid is used.

4. A process as claimed in any of claims 1 to 3, wherein an aqueous sodium carbonate solution is used.

5. A process as claimed in any of claims 1 to 4, wherein the waste liquor is separated off after treatment and incinerated, and the sodium carbonate thus obtained is reused as an aqueous solution for the treatment of cyclohexane oxidation mixtures.

## Revendications

1. Procédé pour le traitement de mélanges réactionnels contenant de l'hydroperoxyde de cyclohexyle, du cyclohexanol et de la cyclohexanone, obtenus par l'oxydation du cyclohexane en phase liquide, à une température comprise entre 130 et 200°C et sous une pression comprise entre 5 et 25 bars, par de l'oxygène moléculaire ou un mélange gazeux contenant de l'oxygène moléculaire, par le traitement à température accrue et en présence de sels de cobalt par des solutions aqueuses de métaux alcalins, caractérisé en ce que l'on utilise simultanément des composés d'acide phosphonique de la formule

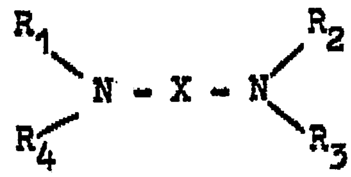

(I),

dans laquelle X représente un groupe alkylène en $C_2$ à $C_6$, $R_1$ et $R_4$ désignent chacun un groupe $-CH_2PO_3H$, tandis que $R_2$ et $R_3$, qui peuvent être identiques ou diffrérents, représentent un groupe $-CH_2PO_3H$ ou $-CH_2OH$ ou un atome d'hydrogène ou un radical alkyle en $C_1$ à $C_4$.

2. Procédé suivant la revendication 1, caractérisé en ce que l'on emploie des sels de cobalt hydrosolubles.

3. Procédé suivant les revendications 1 et 2, caractérisé en ce que l'on utilise de l'acide éthylènediamine-tétraméthylène-phosphonique.

4. Procédé suivant les revendications 1 à 3, caractérisé en ce que l'on emploie une solution aqueuse de carbonate de sodium.

5. Procédé suivant les revendications 1 à 4, caractérisé en ce qu'après le traitement, la lessive résiduaire est séparée et brûlée et le carbonate de sodium récupéré sert comme solution aqueuse pour le traitement des mélanges d'oxydation du cyclohexane.